# EUROPEAN PATENT SPECIFICATION

(11) **EP 2 962 728 B1**
(45) Date of publication and mention of the grant of the patent: **16.01.2019**
(21) Application number: 13876137.4
(22) Date of filing: 28.02.2013
(51) Int. Cl.: A61N 7/02, A61N 7/00, A61B 8/08, A61B 8/14, A61B 18/00, G01S 15/89

(54) **ULTRASONIC MEDICAL APPARATUS FOR FOCAL POINT COMPENSATION**
MEDIZINISCHE ULTRASCHALLVORRICHTUNG ZUR BRENNPUNKTKOMPENSATION
APPAREIL MÉDICAL ULTRASONORE DE COMPENSATION DE FOYER

(43) Date of publication of application: 06.01.2016
(73) Proprietor: Alpinion Medical Systems Co., Ltd., Hwaseong-si, Gyeonggi-do 445-380 (KR)
(72) Inventor: SON, Keonho, Seongnam-si Gyeonggi-do 463-440 (KR); KANG, Kookjin, Yongin-si Gyeonggi-do 448-536 (KR); KIM, Dae Seung, Seoul 157-882 (KR); KIM, Myungdeok, Seoul 152-020 (KR); JUN, Sukhwan, Incheon 402-200 (KR)
(74) Representative: Harden, Henry Simon
(86) International application number: PCT/KR2013/001669
(87) International publication number: WO 2014/133208

(56) References cited:
- WO-A1-2004/075987
- WO-A2-01/80708
- JP-A- H0 759 777
- KR-A- 20100 120 091
- KR-A- 20110 039 506
- KR-A- 20120 010 011
- KR-A- 20120 010 012
- KR-B1- 101 031 504
- DATABASE BIOSIS [Online] BIOSCIENCES INFORMATION SERVICE, PHILADELPHIA, PA, US; 1994, WU REN-GUANG ET AL: "Image comparison of real-time gray-scale ultrasound and color Doppler ultrasound for use in diagnosis of minimal pleural effusion", XP002761637, Database accession no. PREV199497469938 & AMERICAN JOURNAL OF RESPIRATORY AND CRITICAL CARE MEDICINE, vol. 150, no. 2, 1994, pages 510-514,

## Description

### [Technical Field]

The present disclosure relates to an apparatus for compensating a focal point, and more particularly, to a method and an ultrasound medical apparatus for focusing a high-intensity ultrasound having an intensity lower than a predetermined output value on a target object (an affected area of a patient), and compensating for a distance of the focal point by a difference as determined to be present between the focal point on the target object and the actual ultrasound focusing location.

### [Background]

The statements in this section merely provide background information related to the present disclosure and do not necessarily constitute prior art.

A high-intensity focused ultrasound (HIFU) is generally used for treating (processing) a biological tissue such as cancer, tumor, and lesion. The treatment method using a high-intensity ultrasound necrotizes a biological tissue by using a heat generated by transmitting the high-intensity ultrasound to a single spot in a focused manner. In this case, the high-intensity ultrasound needs to be controlled not to damage a healthy biological tissue, and using such a high-intensity focused ultrasound obviates an incision process from a surgical operation.

In an exemplary treatment method using the high-intensity ultrasound, an ultrasound for acquiring an image is transmitted to a biological tissue to be treated, an image is acquired by using an echo signal which is a reflected signal of the ultrasound, and then a high-intensity ultrasound is transmitted to the corresponding biological tissue. In this case, a possible difference between the focal point to be treated by the ultrasound medical apparatus and the actual location where the high-intensity ultrasound is focused causes damage to a biological tissue out of the focal point.

WO 2004/075987 A1 discloses a high intensity focused ultrasound (HIFU) for medically treating tumors that is automatically administered under robotic control in dosage intervals that alternate with ultrasonic imaging intervals.

### [Disclosure]

### [Technical Problem]

The present disclosure has been made in an effort to effectively resolving at least a part of the above-mentioned problems, and it is an object of some embodiments of the present disclosure to provide an ultrasound medical apparatus for focusing a high-intensity ultrasound having an intensity lower than a predetermined output value on a target object or an affected area of a patient, and when it is determined that there is a difference between the focal point on the target object and the actual ultrasound focusing location, compensating for a distance of the focal point by the difference.

### [Summary]

The invention is defined by the appended independent claim. Preferred embodiments of the invention are illustrated in the dependent claims. According to some embodiments of the present disclosure, an ultrasound medical apparatus is provided, which includes an imaging transducer configured to transmit a first imaging ultrasound to a target object, to receive a first echo signal reflected from the target object, and to generate a first reception signal based on the first echo signal, a signal processing unit configured to generate a first image picture based on the first reception signal and to output the first image picture to a display unit, a user input unit configured to receive an input of focal-point information on the first image picture, a treatment transducer configured to transmit a high-intensity ultrasound having an intensity lower than a predetermined output value to a focal point corresponding to the focal-point information, a focusing-location extracting unit configured to extract actual focal-point information by using a change amount of a second image picture that is generated based on a second imaging ultrasound transmitted at a time when the high-intensity ultrasound reaches the focal point, and a compensating unit configured to generate a compensation value based on the focal-point information and the actual focal-point information and to apply the compensation value to the focal-point information.

According to another embodiment of the present disclosure, a method, not according to the present invention, for compensating for a focal point in an ultrasound medical apparatus is provided, which includes:
a first reception signal generating step including transmitting a first imaging ultrasound to a target object, receiving a first echo signal reflected from the target object, and generating a first reception signal based on the first echo signal,
a first image processing step including generating a first image picture based on the first reception signal, and outputting the first image picture to a display unit,
a focal point inputting step including receiving an input of focal-point information on the first image picture,
a high-intensity ultrasound transmitting step including transmitting a high-intensity ultrasound having an intensity lower than a predetermined output value to a focal point corresponding to the focal-point information,
an actual focal-point extracting step including extracting actual focal-point information by using a change amount of a second image picture that is generated based on a second imaging ultrasound transmitted at a time when the high-intensity ultrasound reaches the focal point, and
a compensating step including generating a compensation value based on the focal-point information and the actual focal-point information, and applying the compensation value to the focal-point information.

### [Advantageous Effects]

According to some embodiments of the present disclosure as described above, a pre-targeting can be achieved by focusing a high-intensity ultrasound having an intensity lower than a predetermined output value on a target object or an affected area of a patient and using a reflected signal of the ultrasound, and when an error occurs between the focal point on the target object and the actual ultrasound focusing location, a distance of the focal point can be compensated by as much as the difference. According to some embodiments, a pre-targeting is performed to avail of the correct focus by transmitting an undamaging level of high-intensity ultrasound to a human tissue at the focal point, and a possible mismatch between the focal point on the target object and the actual ultrasound focusing location can be compensated by using an electronic compensation value without a physical movement.

Further, according to some embodiments, off-focusing issues are addressed when the treatment transducer transmits the high-intensity ultrasound for treatment to the set focal point and if there is a mismatch between the focal-point information and the actual focusing location information risking damage to a biological tissue (target object) deviated from the treatment focal-point information, by compensating the difference between the focal-point information and the actual focusing location information, thereby treating the site to be treated in a precise manner.

### [Brief Description of Drawings]

FIG. 1 is a block diagram of an ultrasound medical apparatus according to some embodiments.
FIG. 2 is a schematic diagram of a high-intensity ultrasound having an intensity lower than a predetermined output value according to some embodiments.
FIG. 3 is a schematic diagram for illustrating focal-point information on an image picture according to some embodiments.
FIG. 4 is a flowchart of a method for compensating for a focal point.
FIGs. 5A and 5B are schematic diagrams for illustrating a difference between the focal-point information and the actual focusing location information according to some embodiments.
FIG. 6 is a schematic diagram for illustrating a process of generating a three-dimensional image picture by an ultrasound medical apparatus according to some embodiments.
FIG. 7 is a schematic diagram of a treatment transducer including a plurality of groups according to some embodiments.

**REFERENCE NUMERALS**

| | |
|---|---|
| 100: Ultrasound Medical Apparatus | 110: Imaging Transducer |
| 112: Treatment Transducer | 120: Analog-to-digital Converter |
| 130: Image Processing Unit | 132: Beamformer |
| 134: Signal Processing Unit | 136: Scan Converter |
| 138: Focusing-location Extracting Unit | 139: Compensating Unit |
| 140: Display Unit | 150: User Input Unit |
| 160: Storage Unit | |

### [Detailed Description]

Hereinafter, at least one embodiment of the present disclosure will be described in detail with reference to the accompanying drawings.

A first imaging ultrasound described in some embodiments refers to an ultrasound that is transmitted to a target object to obtain an image by using an imaging transducer 110. On the other hand, a second imaging ultrasound described in some embodiments refers to an ultrasound that is transmitted to the target object by the imaging transducer 110 at a time when a high-intensity ultrasound is transmitted by a treatment transducer 112 and reaches a focal point. When a high-intensity ultrasound having an intensity lower than a predetermined output value is transmitted by the treatment transducer 112 based on a sort of synchronization between the imaging transducer 110 and the treatment transducer 112, the imaging transducer 110 can determine the time when the high-intensity ultrasound reaches the focal point and transmit the second imaging ultrasound to the target object according to the arrival time of the high-intensity ultrasound. Such a synchronization can be set based on a distance information between the imaging transducer 110 and the focal point, distance information between the treatment transducer 112 and the focal point, and traveling speed information of the first imaging ultrasound, the second imaging ultrasound, and the high-intensity ultrasound traveling in the medium of the target object.

A diagnostic image described in some embodiments includes a B-mode image, a C-mode image, and the like. The B-mode image is a grayscale image in an image mode for displaying a motion of a target object, and the C-mode image is an image in a color flow image mode. A BC-mode image is an image in an image mode for displaying a blood flow or a motion of the target object by using a Doppler effect, which simultaneously provides the B-mode image and the C-mode image to provide anatomic information together with the blood flow and the motion of the target object. The B-mode is a grayscale image mode for displaying the motion of the target object, and the C-mode is a color flow image mode for displaying the blood flow or the motion of the target object. An ultrasound medical apparatus 100 according to some embodiments is capable of simultaneously providing the B-mode image and the C-mode image that is a color flow image; however, for the purpose of illustration, descriptions are given with an assumption that a diagnostic image is a B-mode image that is provided by the ultrasound medical apparatus 100 in the present disclosure.

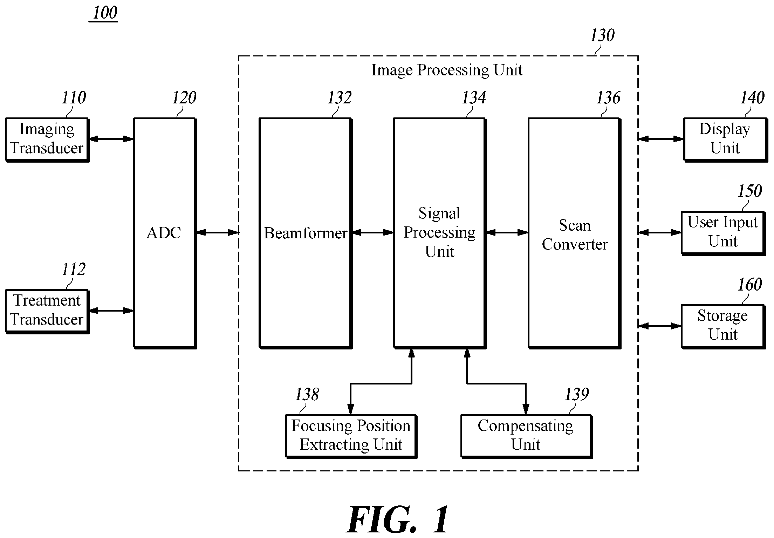

FIG. 1 is a block diagram of an ultrasound medical apparatus according to some embodiments.

An ultrasound medical apparatus 100 includes an imaging transducer 110, a treatment transducer 112, an analog-to-digital converter 120, an image processing unit 130, a display unit 140, a user input unit 150, and a storage unit 160. To illustrate an exemplary technical idea of some embodiments, the ultrasound medical apparatus 100 specifically includes the imaging transducer 110, treatment transducer 112, analog-to-digital converter 120, image processing unit 130, display unit 140, user input unit 150 and storage unit 160 in some embodiments, although one of ordinary skill in the pertinent art would appreciate that various modifications, additions and substitutions are possible in the constituent elements of the ultrasound medical apparatus 100 without departing from the scope of the embodiments.

The imaging transducer 110 includes an imaging transducer array, transmits a first imaging ultrasound and a second imaging ultrasound to a target object, and receives a first echo signal and a second echo signal reflected from the target object. This transducer converts an electrical analog signal into an ultrasound, transmits the ultrasound to the target object, converts the echo signal reflected from the target object into an electrical analog signal, and includes a plurality of transducer elements coupled to each other. This imaging transducer 110 includes one-dimensional (1D), 1.25D, 1.5D, 1.75D, or 2D transducer array. For example, when the imaging transducer 110 includes 1D, 1.25D, 1.5D, or 1.75D transducer array, the 1D, 1.25D, 1.5D, or 1.75D transducer array transmits the first imaging ultrasound or the second imaging ultrasound while rotating in a predetermined angle (0° to 360°), and forms a first reception signal or a second reception signal by receiving the first echo signal or the second echo signal from the target object. On the other hand, when the imaging transducer 110 includes 2D transducer array, the imaging transducer 110 transmits the first imaging ultrasound or the second imaging ultrasound via the 2D transducer array without a rotation, and forms the first reception signal or the second reception signal by receiving the first echo signal or the second echo signal from the target object. The imaging transducer 110 transmits an ultrasound beam, which is focused by appropriately delaying an input time of a pulse inputted to each transducer, to the target object along a transmission scanline. On the other hand, the first echo signal or the second echo signal reflected from the target object is inputted to each transducer with a different reception time, and each transducer outputs the first echo signal or the second echo signal to a beamformer 132.

The imaging transducer 110 transmits the first imaging ultrasound to the target object, receives the first echo signal reflected from the target object, and forms the first reception signal based on the first echo signal. In some embodiments, the imaging transducer 110 further transmits the second imaging ultrasound to the target object at a time when the high-intensity ultrasound reaches the focal point, receives the second echo signal reflected in response to the second imaging ultrasound, and forms the second reception signal based on the second echo signal. When a high-intensity ultrasound having an intensity lower than a predetermined output value reaches the focal point, although a human tissue corresponding to the focal point is not damaged, the corresponding human tissue is depressed or a density of the human tissue is changed by the high-intensity ultrasound having the intensity lower than the predetermined output value. The second imaging ultrasound is transmitted to the target object at the time when the high-intensity ultrasound reaches the focal point, and then reflected second echo signal is received, which can detect the spot where the human tissue is depressed or the density of the human tissue is changed by the high-intensity ultrasound having the intensity lower than the predetermined output value. In other words, when the focal point is irradiated with an acoustic pressure having a high amplitude (high-intensity ultrasound), the medium is instantly depressed to cause a change of density and wave velocity, and the ultrasound is reflected due to a difference in the acoustic impedance. In order to determine the time when the high-intensity ultrasound reaches the focal point by the imaging transducer 110, when the high-intensity ultrasound having the intensity lower than the predetermined output value is transmitted by the treatment transducer 112 under a sort of synchronization between the imaging transducer 110 and the treatment transducer 112, the imaging transducer 110 transmits the second imaging ultrasound to the target object according to the arrival time of the high-intensity ultrasound. Such a synchronization can be set based on the distance information between the imaging transducer 110 and the focal point, the distance information between the treatment transducer 112 and the focal point, and the traveling speed information of the first imaging ultrasound, the second imaging ultrasound, and the high-intensity ultrasound traveling in the medium of the target object.

The treatment transducer 112 includes a high-intensity transducer array, and transmits a high-intensity ultrasound having an intensity lower than a predetermined output value or a high-intensity ultrasound for treatment (corresponding to the predetermined output value) to a specific region of the target object. The treatment transducer 112 transmits the high-intensity ultrasound having the intensity lower than the predetermined output value to a focal point corresponding to focal-point information. The treatment transducer 112 transmits a high-intensity ultrasound having a duty ratio lower than a predetermined ratio and a pulse amplitude exceeding a predetermined threshold value to the target object. For example, the treatment transducer 112 transmits the high-intensity ultrasound for treatment and the high-intensity ultrasound for pre-targeting. That is, the treatment transducer 112 transmits the high-intensity ultrasound for pre-targeting with a low intensity (with the duty ratio lower than the predetermined ratio, e.g., equal to or smaller than 5%) not to damage the human tissue (target object) or transmits the high-intensity ultrasound with a high intensity (with the duty ratio of the predetermined ratio of, e.g., 100%). At this time, the treatment transducer 112 repeatedly transmits the high-intensity ultrasound having the intensity lower than the predetermined output value for predetermined times. For example, the treatment transducer 112 makes a test pre-targeting by performing about two repeat transmissions of the high-intensity ultrasound having the intensity lower than the predetermined output value to the target object. In some embodiments, the two repeat transmissions are good for the high-intensity ultrasound having the intensity lower than the predetermined output value; however, the present disclosure is not limited thereto.

An exemplary operation of the treatment transducer 112 is described below. The treatment transducer 112 transmits the high-intensity ultrasound (one having the intensity lower than the predetermined output value or having the intensity corresponding to the predetermined output value) to a specific site determined via the user input unit 150. In this case, the user first transmits the first imaging ultrasound to the target object via the imaging transducer 110, receives the first echo signal reflected from the target object, and determines the specific site (focal point corresponding to the focal-point information) of the target object through a first image picture generated based on the first reception signal obtained upon receiving the first echo signal. In order for the user to determine the specific site (focal point corresponding to the focal-point information), the user inputs a location value corresponding to the specific site via the user input unit 150 or determines the corresponding site by using a direction key such as a joystick. With this operation, the high-intensity ultrasound can be transmitted to the specific site of the target object, such as cancer tissue, tumor tissue, lesion tissue, or the like. In some embodiments, the treatment transducer 112 is manufactured in a circular shape with the imaging transducer 110 arranged at the center; however, the present disclosure is not limited to this scheme.

In some embodiments, the treatment transducer 112 is divided into a plurality of groups. The treatment transducer 112 includes a high-intensity transducer array which is divided into a first transducer that transmits a first high-intensity ultrasound having an intensity lower than a predetermined output value based on a fundamental frequency to the specific site of the target object, a second transducer that transmits a second high-intensity ultrasound having an intensity lower than a predetermined output value based on a second frequency to the specific site of the target object, and an N-th transducer that transmits an N-th high-intensity ultrasound having an intensity lower than a predetermined output value based on an N-th frequency to the specific site of the target object. The treatment transducer 112 includes a first group including at least one first transducer, a second group including at least one second transducer, and an N-th group including at least one N-th transducer. Further, in some embodiments, the treatment transducer 112 includes the first transducer, the second transducer, and the N-th transducer arranged in a random manner. In this case, the treatment transducer 112 transmits a combination of a plurality of high-intensity ultrasounds with a ratio loaded to the specific region of the target object, or transmits the high-intensity ultrasound having the intensity lower than the predetermined output value by using each of the groups.

The analog-to-digital converter 120 converts the electrical analog signal obtained by the transducer of the imaging transducer 110 or the treatment transducer 112 into an electrical digital signal. FIG. 1 shows that the analog-to-digital converter 120 is arranged between the transducer (the imaging transducer 110 or the treatment transducer 112) and the beamformer 132, although the present invention is not limited to this scheme. One of ordinary skill in the pertinent art would appreciate that various modifications are possible in the arrangement of the analog-to-digital converter 120 between other modules in the ultrasound medical apparatus 100 without departing from the idea and scope of the embodiments.

The image processing unit 130 generates a first image picture by using the first reception signal generated based on the first echo signal corresponding to the first imaging ultrasound from the imaging transducer 110. Further, the image processing unit 130 generates a second image picture by using the second reception signal generated based on the second echo signal corresponding to the second imaging ultrasound from the imaging transducer 110.

Further, the image processing unit 130 is configured to set a region of interest (ROI) on the first image picture by an operation or an instruction from the user input unit 150. The image processing unit 130 receives the first echo signal from an image transducer that is rotated by a predetermined angle (0° to 360°) or from the imaging transducer 110 that includes a two-dimensional (2D) transducer array, and forms the first image picture as a three-dimensional image based on the received first echo signal. Further, the image processing unit 130 receives the second echo signal from the imaging transducer that is rotated by a predetermined angle (0° to 360°) or from the imaging transducer 110 that includes a 2D transducer array, and forms the second image picture as a three-dimensional image based on the received second echo signal.

The image processing unit 130 includes a beamformer 132, a signal processing unit 134, a scan converter 136, a focusing-location extracting unit 138, and a compensating unit 139. The beamformer 132 focuses the first echo signal and the second echo signal received by each transducer of the imaging transducer 110, and generates frame data as raw data. The beamformer 132 generates a receive focusing signal based on the electrical digital signal obtained by the analog-to-digital converter 120. Further, the beamformer 132 is configured to generate the receive focusing signal by adding an appropriate delay to each electrical digital signal and then summing the electrical digital signals, considering a time required to reach each transducer of the imaging transducer 110 and the treatment transducer 112 from the target object. The beamformer 132 focuses the ultrasound on a focal point (specific site) corresponding to the focal-point information by adjusting driving timing of each transducer in the imaging transducer 110 and the treatment transducer 112 when the imaging transducer 110 transmits the first imaging ultrasound or the second imaging ultrasound or the treatment transducer 112 transmits the high-intensity ultrasound. Further, the beamformer 132 focuses the first echo signal and the second echo signal by adding a time delay to each of the first imaging ultrasound signal and the second imaging ultrasound signal of the imaging transducer 110, considering the difference in time required for the first echo signal and the second echo signal reflected at the target subject to reach each transducer of the imaging transducer 110. The beamformer 132 in its implementation includes an imaging beamformer corresponding to the imaging transducer 110 and a treatment beamformer corresponding to the treatment transducer 112, and each of the imaging beamformer and the treatment beamformer includes a transmit beamformer and a receive beamformer.

The signal processing unit 134 generates the first image picture or the second image picture by performing a digital signal processing on the frame data signal generated by the beamformer 132. That is, the signal processing unit 134 generates the first image signal based on the first reception signal received from the imaging transducer 110, and outputs the first image picture to the display unit 140. Further, the signal processing unit 134 generates the second image signal based on the second reception signal received from the imaging transducer 110, and outputs the second image picture to the display unit 140. The scan converter 136 converts a data format of the first image picture or the second image picture into a data format that can be used in the display unit 140 of a predetermined scanline display format. That is, the scan converter 136 converts the first image picture signal or the second image picture signal into a signal of a data format that can be actually displayed on the display unit 140.

The focusing-location extracting unit 138 according to some embodiments extracts actual focusing-location information by using a change amount of the second image picture generated based on the second imaging ultrasound transmitted at the time when the high-intensity ultrasound reaches the focal point. That is, the imaging transducer 110 transmits the second imaging ultrasound at the time when the high-intensity ultrasound having the intensity lower than the predetermined output value transmitted by the treatment transducer 112 reaches the focal point, receives the second echo signal reflected in response to the second imaging ultrasound, and generates the second reception signal based on the second echo signal. Thereafter, the signal processing unit 134 generates the second image picture based on the second reception signal, and at this time, the focusing-location extracting unit 138 extracts the actual focusing-location information by using the change amount of the second image picture. In order to detect the change amount of the second image picture, the focusing-location extracting unit 138 extracts a location having the largest change amount of gray-scale of the second image picture, and determines the location having the largest change amount as the actual focusing-location information. The gray-scale is an index of brightness difference, which is a gradual step ranging from white to black. In a digital image, each pixel value indicates a single sample image, and the digital image delivers luminous intensity information only. When the second image picture is a two-dimensional image, the focusing-location extracting unit 138 determines two-dimensional coordinates as the actual focusing-location information (x₁, z₁), and when the second image picture is a three-dimensional image, it determines three-dimensional coordinates as the actual focusing-location information (x₁, y₁, z₁).

The compensating unit 139 according to some embodiments extracts a compensation value based on the focal-point information and the actual focusing-location information, and then applies the compensation value to the focal-point information. When the second image picture is a two-dimensional image, the compensating unit 139 generates the compensation value (Δx, Δz) based on a difference (x - x₁, z - z₁) between the focal-point information (x, z) on two-dimensional coordinates and the actual focal-point information (x₁, z₁) on the two-dimensional coordinates, and when the second image picture is a three-dimensional image, it generates the compensation value (Δx, Δy, Δz) based on a difference (x - x₁, y - y₁, z - z₁) between the focal-point information (x, y, z) on three-dimensional coordinates and the actual focal-point information (x₁, y₁, z₁) on the three-dimensional coordinates. Further, when the second image picture is a two-dimensional image, the compensating unit 139 generates compensation location information (x + Δx, z + Δz) by applying the compensation value (Δx, Δz) to the focal-point information (x, z) on the two-dimensional coordinates, and when the second image picture is a three-dimensional image, it generates compensation location information (x + Δx, y + Δy, z + Δz) by applying the compensation value (Δx, Δy, Δz) to the focal-point information (x, y, z) on the three-dimensional coordinates. That is, when the treatment transducer 112 transmits the high-intensity ultrasound for treatment with the focal-point information set by the user input unit 150, a possible difference between the focal-point information (x, y, z) and the actual focusing-location information (x₁, y₁, z₁) risks damage to a biological tissue (target object) deviated from the focal-point information to be treated may be damaged. Therefore, the compensating unit 139 according to some embodiments detects the difference between the focal-point information (x, y, z) and the actual focusing-location information (x₁, y₁, z₁), and compensates for the distance (Δx, Δy, Δz) by the difference, thus accurately treating the site at issue.

The display unit 140 outputs the first image picture or the second image picture received from the image processing unit 130 as a B-mode image or a C-mode image. The user input unit 150 receives an instruction by an operation or an input of a user. The user instruction includes a control instruction to control the ultrasound medical apparatus 100 and the like. Further, the user input unit 150 receives an input of the focal-point information on the first image picture. That is, when the first image picture is a two dimensional image, the user input unit 150 receives an input of the focal-point information of two-dimensional coordinates (x, z), and when the first image picture is a three-dimensional image, it receives an input of the focal-point information of three-dimensional coordinates (x, y, z). Moreover, the user input unit 150 is configured to set an ROI on the first image picture by an operation or an instruction from the user. The storage unit 160 is for storing various data for driving the ultrasound medical apparatus 100, which stores at least one of the first image picture or the second image picture. Further, the storage unit 160 stores the first reception signal or the second reception signal generated by using the imaging transducer 110.

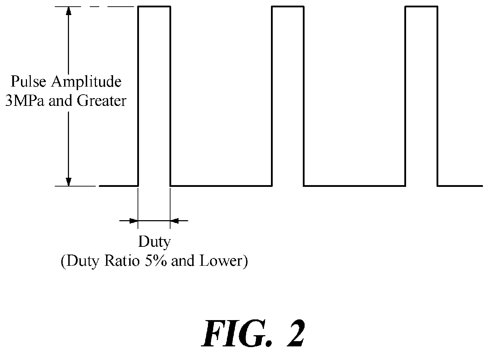

FIG. 2 is a schematic diagram of a high-intensity ultrasound having an intensity lower than a predetermined output value according to some embodiments.

As shown in FIG. 2, the high-intensity ultrasound having the intensity lower than the predetermined output value according to some embodiments has a duty ratio lower than a predetermined ratio and a pulse amplitude exceeding a predetermined threshold value. That is, the treatment transducer 112 is configured to transmit a high-intensity ultrasound for treatment and another high-intensity ultrasound for pre-targeting. The treatment transducer 112 transmits the high-intensity ultrasound for pre-targeting with a low intensity (with the duty ratio lower than the predetermined ratio, e.g., equal to or smaller than 5%) not to damage the human tissue (target object). On the other hand, the treatment transducer 112 transmits the high-intensity ultrasound with a high intensity (with the duty ratio of the predetermined ratio of, e.g., 100%) to treat the human tissue. That is, in the example shown in FIG. 2, the treatment transducer 112 transmits the high-intensity ultrasound for pre-targeting with a low intensity (with the duty ratio lower than the predetermined ratio, e.g., equal to or smaller than 5%) not to damage the human tissue (target object). At this time, the treatment transducer 112 transmits the high-intensity ultrasound with a low intensity (with the duty ratio lower than the predetermined ratio, e.g., equal to or smaller than 5%) not to damage the human tissue (target object) in a repeated manner for a predetermined number of times to a specific site of the target object (focal point corresponding to the focal-point information). That is, the treatment transducer 112 performs a number of (about two) repeat transmissions of the high-intensity ultrasound having the intensity lower than the predetermined output value to the target object, thus performing the pre-targeting.

As shown in FIG. 2, in some embodiments, the high-intensity ultrasound that is set as low as not to damage the human tissue (target object) (to have a duty ratio lower than a predetermined ratio (e.g., equal to or smaller than about 5%) has a pulse amplitude of equal to or larger than about 3 MPa; however, the present invention is not limited to this scheme. The unit MPa is a unit of acoustic pressure, and the acoustic pressure can be represented as an acoustic Intensity (unit: W/cm²). That is, the acoustic pressure of 3 MPa can be converted into the acoustic intensity of 580 W/cm². When treating the human tissue (target object), energy is equal to power x time, and hence in order to reduce the damage, the time should be decreased. To this end, the duty ratio is set to be small (e.g., equal to or smaller than about 5%).

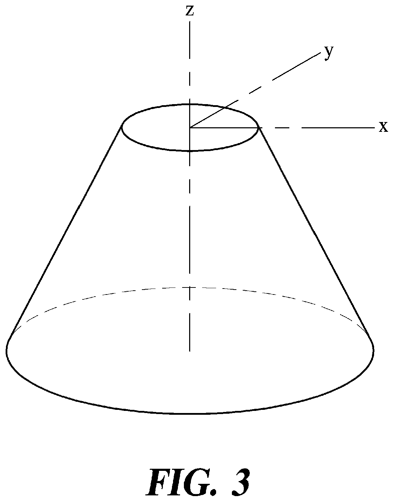

FIG. 3 is a schematic diagram for illustrating focal-point information on an image picture according to some embodiments.

In some embodiments, the ultrasound medical apparatus 100 transmits the first imaging ultrasound or the second imaging ultrasound to the target object, receives the first echo signal or the second echo signal reflected from the target object, generates the first reception signal or the second reception signal based on the first echo signal or the second echo signal, generates the first image picture or the second image picture based on the first reception signal or the second reception signal, and then outputs the first image picture or the second image picture to the display unit 140. In some embodiments, the first image picture or the second image picture generated by the ultrasound medical apparatus 100 is a three-dimensional image; however, the present invention is not limited to this scheme. When the imaging transducer 110 in the ultrasound medical apparatus 100 includes a 2D transducer array, the ultrasound medical apparatus 100 generates the first image picture as a three-dimensional image based on the first echo signal received corresponding to the first imaging ultrasound transmitted by the 2D transducer array. On the other hand, when the imaging transducer 110 in the ultrasound medical apparatus 100 includes a 2D transducer array, the ultrasound medical apparatus 100 generates the second image picture as a three-dimensional image based on the second echo signal received corresponding to the second imaging ultrasound transmitted by the 2D transducer array.

However, when the imaging transducer 110 in the ultrasound medical apparatus 100 includes a 1D, 1.25D, 1.5D and 1.75D transducer arrays, the ultrasound medical apparatus 100 transmits the first imaging ultrasound or the second imaging ultrasound while rotating the 1D, 1.25D, 1.5D and 1.75D transducer arrays by a predetermined angle (0° to 360°), receives the first echo signal or the second echo signal reflected from the target object, and then generates the first image picture or the second image picture based on the first echo signal or the second echo signal. As described above, when the first image picture or the second image picture is a three-dimensional image, as shown in FIG. 3, the coordinates can be represented as (x, y, z). When the imaging transducer 110 includes a 1D, 1.25D, 1.5D and 1.75D transducer arrays, the first image picture or the second image can be generated as a two-dimensional image, and in this case, the coordinates can be represented as (x, z).

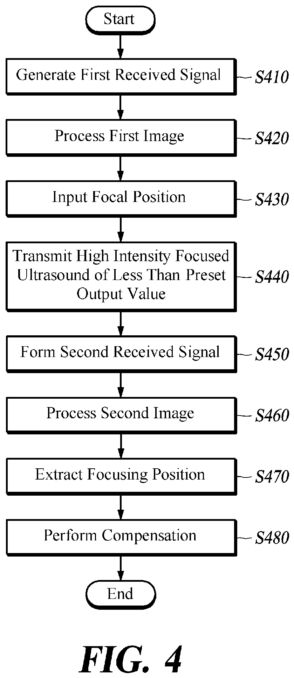

FIG. 4 is a flowchart of a method of compensating for a focal point.

The imaging transducer 110 of the ultrasound medical apparatus 100 transmits the first imaging ultrasound to the target object, receives the first echo signal reflected from the target object, and generates the first reception signal based on the first echo signal (step S410). The signal processing unit 134 of the ultrasound medical apparatus 100 generates the first image picture based on the first reception signal, and outputs the first image picture to the display unit 140 (step S420).

The user input unit 150 of the ultrasound medical apparatus 100 receives an input of the focal-point information on the first image picture (step S430). At step S430, when the first image picture is a two-dimensional image, the user input unit 150 receives the focal-point information of two-dimensional coordinates (x, z), and when the first image picture is a three-dimensional image, it receives the focal-point information of three-dimensional coordinates (x, y, z).

The treatment transducer 112 of the ultrasound medical apparatus 100 transmits the high-intensity ultrasound having the intensity lower than the predetermined output value to the focal point corresponding to the focal-point information (step S440). The high-intensity ultrasound having the predetermined output value has a duty ratio lower than a predetermined ratio and a pulse amplitude exceeding a predetermined threshold value. That is, the high-intensity ultrasound having the intensity lower than the predetermined output value means a high-intensity ultrasound having an intensity as low as not to damage the human tissue (target object) or having a duty ratio lower than a predetermined ratio, e.g., equal to or smaller about 5%.

The imaging transducer 110 of the ultrasound medical apparatus 100 transmits the second imaging ultrasound to the target object at the time when the high-intensity ultrasound from the treatment transducer 112 reaches the focal point, receives the second echo signal reflected in response to the second imaging ultrasound, and generates the second reception signal based on the second echo signal (step S450). The signal processing unit 134 of the ultrasound medical apparatus 100 generates the second image picture based on the second reception signal (step S460).

The focusing-location extracting unit 138 of the ultrasound medical apparatus 100 extracts the actual focusing-location information by using the change amount of the second image picture (step S470). At step S470, the focusing-location extracting unit 138 of the ultrasound medical apparatus 100 extracts a location having the largest change amount of the gray-scale of the second image picture, and determines the location having the largest change amount as the actual focusing-location information. At this time, when the second image picture is a two-dimensional image, the focusing-location extracting unit 138 of the ultrasound medical apparatus 100 determines the two-dimensional coordinates as the actual focusing-location information (x₁, z₁), and when the second image picture is a three-dimensional image, it determines the three-dimensional coordinates as the actual focusing-location information (x₁, y₁, z₁).

The compensating unit 139 of the ultrasound medical apparatus 100 extracts a compensation value based on the focal-point information and the actual focusing-location information, and then applies the compensation value to the focal-point information (step S480). At step S480, when the second image picture is a two-dimensional image, the compensating unit 139 of the ultrasound medical apparatus 100 generates the compensation value (Δx, Δz) based on a difference (x - x₁, z - z₁) between the focal-point information (x, z) on two-dimensional coordinates and the actual focal-point information (x₁, z₁) on the two-dimensional coordinates, and when the second image picture is a three-dimensional image, it generates the compensation value (Δx, Δy, Δz) based on a difference (x - x₁, y - y₁, z - z₁) between the focal-point information (x, y, z) on three-dimensional coordinates and the actual focal-point information (x₁, y₁, z₁) on the three-dimensional coordinates. Thereafter, when the second image picture is a two-dimensional image, the compensating unit 139 of the ultrasound medical apparatus 100 generates compensation location information (x + Δx, z + Δz) by applying the compensation value (Δx, Δz) to the focal-point information (x, z) on the two-dimensional coordinates, and when the second image picture is the three-dimensional image, it generates compensation location information (x + Δx, y + Δy, z + Δz) by applying the compensation value (Δx, Δy, Δz) to the focal-point information (x, y, z) on the three-dimensional coordinates.

Steps S410 to S480 are described to be sequentially performed in FIG. 4 as a mere example for describing the technical idea of some embodiments, although one of ordinary skill in the pertinent art would appreciate that various modifications, additions and substitutions are possible by performing the sequences shown in FIG. 4 in a different order or at least one of Steps S410 to S480 in parallel without departing from the idea and scope of the embodiments, and hence the example shown in FIG. 4 is not limited to the chronological order.

The method of compensating the focal point shown in FIG. 4 can be implemented as a computer program, and can be recorded on a computer-readable medium. The computer-readable recording medium on which the method of compensating the focal point according to some embodiments is recordable includes any type of recording device on which data that can be read by a computer system are recordable. Examples of the computer-readable recording medium include a ROM, a RAM, a CD-ROM, a magnetic tape, a floppy disk, an optical data storage device, and the like, and also include one implemented in the form of carrier wave (e.g., transmission through the Internet). Further, the computer-readable recording medium can be distributed in computer systems connected via a network, and computer-readable codes can be stored and executed in a distributed mode. Moreover, functional programs, codes, and code segments for implementing some embodiments can be easily deduced by a programmer in technical fields to which some embodiments belong.

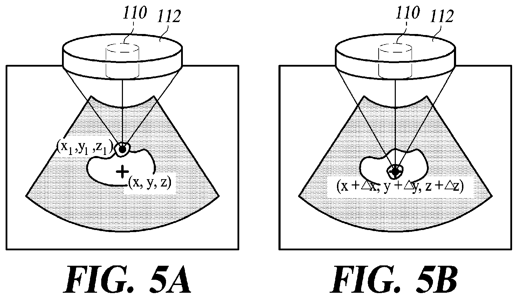

FIGs. 5A and 5B are schematic diagrams for illustrating a difference between the focal-point information and the actual focusing location information according to some embodiments.

As shown in FIGs. 5A and 5B, the user input unit 150 of the ultrasound medical apparatus 100 receives an input of the focal-point information on the first image picture. When the first image picture is a three-dimensional image, the user input unit 150 receives an input of the focal-point information of three-dimensional coordinates (x, y, z). As shown in FIGs. 5A and 5B, a location corresponding to the three-dimensional coordinates (x, y, z) is indicated by "+" on the first image picture. The treatment transducer 112 of the ultrasound medical apparatus 100 transmits the high-intensity ultrasound having the intensity lower than the predetermined output value to the focal point ("+" point shown in FIG. 5A) corresponding to the focal-point information of the target object. Here, the ultrasound medical apparatus 100 transmits the high-intensity ultrasound having the intensity lower than the predetermined output value to a subject to be treated (target object) by using the treatment transducer 112, to increase the reflected signal (second echo signal). That is, when the focal point is irradiated with an acoustic pressure with a large amplitude (high-intensity ultrasound), the medium is instantly depressed to cause changes of density and wave velocity, and hence the ultrasound is reflected due to a difference in the acoustic impedance.

Thereafter, the imaging transducer 110 of the ultrasound medical apparatus 100 transmits the second imaging ultrasound to the target object at the time when the high-intensity ultrasound having the intensity lower than the predetermined output value reaches the focal point. The imaging transducer 110 of the ultrasound medical apparatus 100 receives the second echo signal reflected in response to the second imaging ultrasound, and generates the second reception signal. The signal processing unit 134 of the ultrasound medical apparatus 100 generates the second image picture based on the second reception signal. The focusing-location extracting unit 138 of the ultrasound medical apparatus 100 extracts the actual focusing-location information (location indicated by "●" in FIG. 5A) by using a change amount of the second image picture.

The focusing-location extracting unit 138 of the ultrasound medical apparatus 100 extracts a location having the largest change amount of the gray-scale of the second image picture (location indicated by "●" in FIG. 5A), and determines the location having the largest change amount as the actual focusing-location information (x₁, y₁, z₁). The ultrasound medical apparatus 100 extracts the location having the largest change amount of the gray-scale (location indicated by "●" in FIGs. 5A and 5B) in the ROI set on the second image picture by using the focusing-location extracting unit 138, to determine the actual focusing-location information (x₁, y₁, z₁).

The compensating unit 139 of the ultrasound medical apparatus 100 extracts the compensation value based on the focal-point information (location indicated by "+" in FIG. 5A) and the actual focusing-location information (location indicated by "●" in FIG. 5A), and then applies the compensation value to the focal-point information. Thereafter, as shown in FIG. 5B, when the second image picture is a three-dimensional image, the compensating unit 139 of the ultrasound medical apparatus 100 generates the compensation value (Δx, Δy, Δz) based on a difference (x - x₁, y - y₁, z - z₁) between the focal-point information (x, y, z) on three-dimensional coordinates and the actual focal-point information (x₁, y₁, z₁) on the three-dimensional coordinates. As shown in FIG. 5B, when the second image picture is a three-dimensional image, the compensating unit 139 of the ultrasound medical apparatus 100 generates compensation location information (x + Δx, y + Δy, z + Δz) by applying the compensation value (Δx, Δy, Δz) to the focal-point information (x, y, z) on the three-dimensional coordinates.

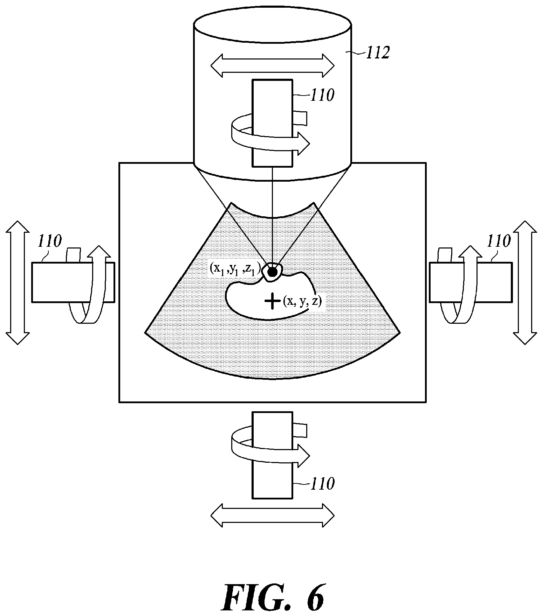

FIG. 6 is a schematic diagram for illustrating a process of generating a three-dimensional image picture by an ultrasound medical apparatus according to some embodiments.

In some embodiments, the ultrasound medical apparatus 100 transmits the first imaging ultrasound or the second imaging ultrasound to the target object, receives the first echo signal or the second echo signal reflected from the target object, generates the first reception signal or the second reception signal based on the first echo signal or the second echo signal, generates the first image picture or the second image picture based on the first reception signal or the second reception signal, and then outputs the first image picture or the second image picture to the display unit 140.

In some embodiments, the first image picture or the second image picture generated by the ultrasound medical apparatus 100 is a three-dimensional image; however, the present disclosure is not limited to this scheme. When the imaging transducer 110 of the ultrasound medical apparatus 100 includes a two-dimensional transducer array, the ultrasound medical apparatus 100 receives the first echo signal corresponding to the first imaging ultrasound transmitted by the 2D transducer array, and generates the first image picture as a three-dimensional image based on the first echo signal, or receives the second echo signal corresponding to the second imaging ultrasound, and generates the second image picture as a three-dimensional image based on the second echo signal. However, when the imaging transducer 110 of the ultrasound medical apparatus 100 includes a 1D, 1.25D, 1.5D and 1.75D transducer arrays, the ultrasound medical apparatus 100 can generate a three-dimensional image in a manner shown in FIG. 3.

As shown in FIG. 6, the ultrasound medical apparatus 100 transmits the first imaging ultrasounds to the target object while rotating the 1D, 1.25D, 1.5D and 1.75D transducer arrays by a predetermined angle (0° to 360°), receives the respective first echo signals reflected from the target object, and then generates the first image picture as a three-dimensional image based on the respective first echo signals, or transmits the second imaging ultrasounds to the target object, receives the respective second echo signals reflected from the target object, and then generates the second image picture as a three-dimensional image based on the second echo signal. For example, the first imaging ultrasounds or the second ultrasounds are transmitted to the target object by using the imaging transducer 110 in a horizontal direction and a vertical direction with respect to the treatment transducer 112, and based on the first echo signals or the second echo signals reflected and received from the target object, the first image picture or the second image picture can be generated as a three-dimensional image.

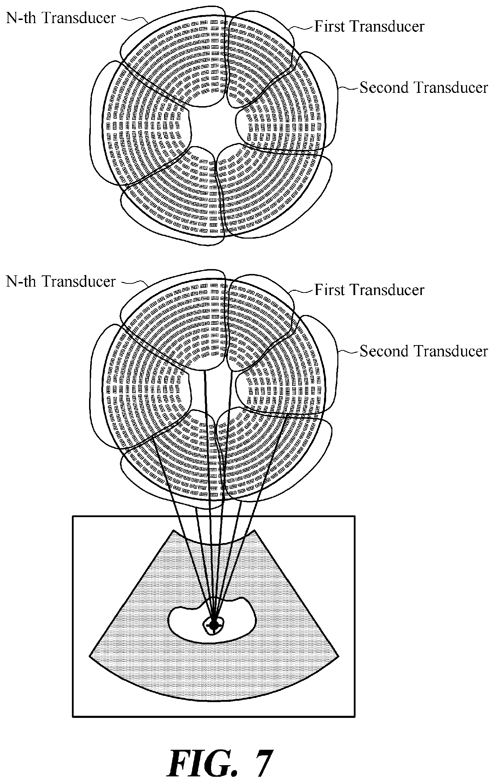

FIG. 7 is a schematic diagram of a treatment transducer including a plurality of groups according to some embodiments.

To take account of the nonuniform internal tissues of a human body (target object), which cause variation of the phase of each acoustic wave reaching the set focal point through different human tissues, the high-intensity transducer array of the treatment transducer 112 can be divided into a plurality (at least two or more) groups. The ultrasound medical apparatus 100 utilizes the treatment transducer 112 to transmit the high-intensity ultrasound having the intensity lower than the predetermined output value to the subject to be treated (target object).

A plurality of grouped divisions of the high-intensity transducer array can also be used successfully to transmit the high-intensity ultrasound having the intensity lower than the predetermined output value to the subject to be treated (target object) and then receive the second echo signals before harming the subject to be treated (human tissue). The ultrasound medical apparatus 100 compensates for the phase by applying an optimization algorithm to the plurality of groups. In this case, the applicable phase is one that occurs when the reflected signal is maximized out of the focal point information, and the applicable optimum solution may be one among a set of various solutions for use as the optimization algorithm.

The treatment transducer 112 is described below with reference to FIG. 7. As shown in FIG. 7, in the treatment transducer 112, the transducers are arranged to form a specific group depending on the first high-intensity ultrasound having an intensity lower than a predetermined output value, the second high-intensity ultrasound having an intensity lower than the predetermined output value, and the N-th high-intensity ultrasound having an intensity lower than the predetermined output value to be transmitted to the focal point corresponding to the focal-point information or arranged in a random manner. The treatment transducer 112 transmits a combination of a plurality of high-intensity ultrasounds with a ratio loaded to the specific region of the target object, or transmits the high-intensity ultrasound having the intensity lower than the predetermined output value by using each of the groups.

Further, the treatment transducer 112 transmits the high-intensity ultrasound to a specific site (the focal point corresponding to the focal-point information) adjusted via the user input unit 150. A user (i.e., an operator) first transmits the first imaging ultrasound to the target object by using the imaging transducer 110, and determines a specific region of the target object through the first image picture generated based on the first reception signal generated based on the first echo signal reflected from the target object. In order for the user (operator) to determine the specific region, the user inputs a location value corresponding to the specific region to the user input unit 150 or determines the corresponding location by adjusting a direction key such as a joystick. With this operation, the high-intensity ultrasound having the intensity lower than the predetermined output value can be transmitted to the specific region of the target object, such as cancer tissue, tumor tissue, or lesion tissue. As shown in FIG. 7, the treatment transducer 112 can be manufactured in a circular shape with the imaging transducer 110 arranged at the center; however, the present invention is not limited to this scheme.

The treatment transducer 112 transmits a combination of a plurality of high-intensity ultrasounds with a ratio loaded to the specific site of the target object under a control of the control unit. As shown in FIG. 7, the treatment transducer 112 includes a first transducer that transmits a first high-intensity ultrasound having an intensity lower than a predetermined output value based on a fundamental frequency to the specific site of the target object, a second transducer that transmits a second high-intensity ultrasound having an intensity lower than a predetermined output value based on a second frequency to the specific site of the target object, and an N-th transducer that transmits an N-th high-intensity ultrasound having an intensity lower than a predetermined output value based on an N-th frequency to the specific site of the target object. The first transducer, the second transducer, and the N-th transducer are physically similar transducers; however, in some embodiments, they are distinguishably described as the first transducer, the second transducer, and the N-th transducer for the purpose of illustration.

More specifically, as shown in FIG. 7, the treatment transducer 112 includes a first group including the first transducers, a second group including the second transducers, and an N-th group including the N-th transducers. Further, in some embodiments, as shown in FIG. 7, the treatment transducer 112 includes the first transducers, the second transducers, and the N-th transducers arranged in a random manner.

Although exemplary embodiments of the present disclosure have been described for illustrative purposes, those skilled in the art will appreciate that various modifications, additions and substitutions are possible, without departing from the scope of the claimed invention. Therefore, exemplary embodiments of the present disclosure have been described for the sake of brevity and clarity. Accordingly, one of ordinary skill in the pertinent art would understand the scope of the claimed invention is not to be limited by the explicitly described above embodiments but by the claims and equivalents thereof.

## Claims

1. An ultrasound medical apparatus (100), comprising:
an imaging transducer (110) configured to:
transmit a first imaging ultrasound to a target object,
receive a first echo signal reflected from the target object in response to the first imaging ultrasound,
generate a first reception signal based on the first echo signal,
transmit a second imaging ultrasound to the target object,
receive a second echo signal reflected from the target object in response to the second imaging ultrasound, and
generate a second reception signal based on the second echo signal;
a signal processing unit (134) configured to:
generate a first image picture based on the first reception signal,
output the first image picture to a display unit, and
generate a second image picture based on the second reception signal;
a user input unit (150) configured to receive an input of focal-point information on the first image picture;
a treatment transducer (112) configured to transmit a first high-intensity ultrasound for pre-targeting having an intensity lower than a predetermined output value and to transmit a second high-intensity ultrasound for treatment having an intensity corresponding to the predetermined output value to a focal point corresponding to the focal-point information;
wherein the imaging transducer and the treatment transducer are synchronized such that the imaging transducer is configured to transmit the second imaging ultrasound to the target object when the first high-intensity ultrasound reaches the focal point, such that the second image picture reflects change in density of the target object as a result of the first high-intensity ultrasound;
a focusing-location extracting unit (138) configured to:
extract from the second image picture a location having a largest change in graysacle relative to the first image picture, and
wherein the extracted location is actual focal-point information representing an actual focal point at which the first high-intensity ultrasound is focused; and
a compensating unit (139) configured to:
generate a compensation value based on the focal-point information and the actual focal-point information, and
apply the compensation value to the focal-point information.

2. The ultrasound medical apparatus according to claim 1, wherein the treatment transducer is configured to transmit the first high-intensity ultrasound having a duty ratio lower than a predetermined ratio and a pulse amplitude exceeding a predetermined threshold value.

3. The ultrasound medical apparatus according to claim 2, wherein the treatment transducer is configured to perform a predetermined repeat transmissions of the first high-intensity ultrasound.

4. The ultrasound medical apparatus according to claim 1, wherein the signal processing unit is configured
to receive the first echo signal from the imaging transducer that is rotated by a predetermined angle in a range from 0 degrees to 360 degrees, or
to receive the first echo signal from the imaging transducer including a two-dimensional transducer array, and
to generate the first image picture as a three-dimensional image based on the first echo signal.

5. The ultrasound medical apparatus according to claim 1, wherein the focusing-location extracting unit is configured
to determine, when the second image picture is a two-dimensional image, two-dimensional coordinates of the location having the largest change amount of the gray-scale of the second image picture as the actual focal-point information (x₁, z₁), and
to determine, when the second image picture is a three-dimensional image, three-dimensional coordinates of the location having the largest change amount of the gray-scale of the second image picture as the actual focal-point information (x₁, y₁, z₁).

6. The ultrasound medical apparatus according to claim 1, wherein the compensating unit is configured
to generate, when the second image picture is a two-dimensional image, the compensation value (Δx, Δz) based on a difference (x - x₁, z - z₁) between the focal-point information (x, z) on two-dimensional coordinates and the actual focal-point information (x₁, z₁) on the two-dimensional coordinates, and
to generate, when the second image picture is a three-dimensional image, the compensation value (Δx, Δy, Δz) based on a difference (x - x₁, y - y₁, z - z₁) between the focal-point information (x, y, z) on three-dimensional coordinates and the actual focal-point information (x₁, y₁, z₁) on the three-dimensional coordinates.

7. The ultrasound medical apparatus according to claim 6, wherein the compensating unit is configured
to generate, when the second image picture is the two-dimensional image, compensation location information (x + Δx, z + Δz) by applying the compensation value (Δx, Δz) to the focal-point information (x, z) on the two-dimensional coordinates, and
to generate, when the second image picture is the three-dimensional image, compensation location information (x + Δx, y + Δy, z + Δz) by applying the compensation value (Δx, Δy, Δz) to the focal-point information (x, y, z) on the three-dimensional coordinates.

8. The ultrasound medical apparatus according to claim 1, wherein the user input unit is configured
to receive, when the first image picture is a two-dimensional image, an input of the focal-point information of two-dimensional coordinates (x, z), and
to receive, when the first image picture is a three-dimensional image, an input of the focal-point information of three-dimensional coordinates (x, y, z).

9. The ultrasound medical apparatus according to claim 1, wherein the treatment transducer includes:
at least one first transducer configured to transmit a first high-intensity ultrasound having an intensity lower than a predetermined output value with respect to a fundamental frequency to the focal point,
at least one second transducer configured to transmit a second high-intensity ultrasound having an intensity lower than a predetermined output value with respect to a secondary frequency to the focal point, and
at least one N-th transducer configured to transmit an N-th high-intensity ultrasound having an intensity lower than a predetermined output value with respect to an N-th frequency to the focal point.

10. The ultrasound medical apparatus according to claim 9, wherein the treatment transducer includes:
a first group consisting of the first transducers,
a second group consisting of the second transducers, and
an N-th group consisting of the N-th transducers.

11. The ultrasound medical apparatus according to claim 9, wherein the treatment transducer includes the first transducers, the second transducers, and the N-th transducers arranged in a random manner.

## Patentansprüche

1. Medizinische Ultraschallvorrichtung (100), umfassend:
einen bildgebenden Wandler (110), ausgelegt zum
Senden eines ersten bildgebenden Ultraschalls zu einem Zielobjekt,
Empfangen eines ersten, vom Zielobjekt in Reaktion auf den ersten bildgebenden Ultraschall reflektierten Echosignals,
Erzeugen eines ersten Empfangssignals, basierend auf dem ersten Echosignal,
Senden eines zweiten bildgebenden Ultraschalls zum Zielobjekt,
Empfangen eines zweiten, vom Zielobjekt in Reaktion auf den zweiten bildgebenden Ultraschall reflektierten Echosignals,
Erzeugen eines zweiten Empfangssignals, basierend auf dem zweiten Echosignal,
eine Signalverarbeitungseinheit (134), ausgelegt zum
Erzeugen einer ersten Bilddarstellung basierend auf dem ersten Empfangssignal,
Ausgeben der ersten Bilddarstellung zu einer Anzeigeeinheit, und
Erzeugen einer zweiten Bilddarstellung basierend auf dem zweiten Empfangssignal;
eine Benutzereingabeinheit (150), ausgelegt zum Empfangen einer Eingabe mit Brennpunktinformation zur ersten Bilddarstellung;
einen Behandlungswandler (112), ausgelegt zum Senden eines ersten Ultraschalls hoher Intensität für das "Pre-Targeting", aufweisend eine geringere Intensität als ein vorbestimmter Ausgabewert, und zum Senden eines zweiten Ultraschalls hoher Intensität zur Behandlung, aufweisend eine dem vorbestimmten Ausgabewert entsprechende Intensität, zu einem der Brennpunktinformation entsprechenden Brennpunkt;
wobei der bildgebende Wandler und der Behandlungswandler synchronisiert sind, sodass der bildgebende Wandler ausgelegt ist zum Senden des zweiten bildgebenden Ultraschalls zum Zielobjekt, wenn der erste Ultraschall den Brennpunkt erreicht, sodass die zweite Bilddarstellung Änderungen der Dichte des Zielobjekts als Ergebnis des ersten Ultraschalls hoher Intensität wiedergibt;
eine Fokussierpositions-Extraktionseinheit (138), ausgelegt zum
Extrahieren einer Position aus der zweiten Bilddarstellung, aufweisend eine größte Änderung in der Grauskala relativ zur ersten Bilddarstellung, und
wobei die extrahierte Position eine Istwert-Brennpunktinformation ist, die einen Istwert-Brennpunkt wiedergibt, bei dem der erste Ultraschall hoher Intensität fokussiert ist; und
eine Kompensationseinheit (139), ausgelegt zum
Erzeugen eines Kompensationswerts basierend auf der Brennpunktinformation und der Istwert-Brennpunktinformation, und
Anwenden des Kompensationswerts auf die Brennpunktinformation.

2. Medizinische Ultraschallvorrichtung nach Anspruch 1, wobei der Behandlungswandler ausgelegt ist zum Senden eines ersten Ultraschalls hoher Intensität, aufweisend ein Tastverhältnis, das geringer ist als ein vorbestimmtes Verhältnis, und eine Pulsamplitude, die einen vorbestimmten Schwellenwert überschreitet.

3. Medizinische Ultraschallvorrichtung nach Anspruch 2, wobei der Behandlungswandler ausgelegt ist zum Durchführen von vorbestimmten Wiederholungssendungen des ersten Ultraschalls hoher Intensität.

4. Medizinische Ultraschallvorrichtung nach Anspruch 1, wobei die Signalverarbeitungseinheit ausgelegt ist
zum Empfangen des ersten Echosignals vom bildgebenden Wandler, der um einen vorbestimmten Winkel in einem Bereich von 0 Grad bis 360 Grad rotiert wird, oder
zum Empfangen des ersten Echosignals vom bildgebenden Wandler, aufweisend ein zweidimensionales Wandler-Array, und
zum Erzeugen der ersten Bilddarstellung als ein dreidimensionales Bild basierend auf dem ersten Echosignal.

5. Medizinische Ultraschallvorrichtung nach Anspruch 1, wobei die Fokussierpositions-Extraktionseinheit ausgelegt ist
zum Festlegen, wenn die zweite Bilddarstellung ein zweidimensionales Bild ist, von zweidimensionalen Koordinaten der Position, die den größten Änderungsumfang der Grauskala der zweiten Bilddarstellung aufweist, als Istwert-Brennpunktinformation (x₁, z₁), und
zum Festlegen, wenn die zweite Bilddarstellung ein dreidimensionales Bild ist, von dreidimensionalen Koordinaten der Position, die den größten Änderungsumfang der Grauskala der zweiten Bilddarstellung aufweist, als Istwert-Brennpunktinformation (x₁, y₁, z₁).

6. Medizinische Ultraschallvorrichtung nach Anspruch 1, wobei die Kompensationseinheit ausgelegt ist
zum Erzeugen, wenn die zweite Bilddarstellung ein zweidimensionales Bild ist, des Kompensationswerts (Δx, Δz), basierend auf einer Differenz (x - x₁, z - z₁) zwischen der Brennpunktinformation (x, z) an zweidimensionalen Koordinaten und der Istwert-Brennpunktinformation (x₁, z₁) an den zweidimensionalen Koordinaten, und
zum Erzeugen, wenn die zweite Bilddarstellung ein dreidimensionales Bild ist, des Kompensationswerts (Δx, Δy, Δz), basierend auf einer Differenz (x - x₁, y - y₁, z - z₁) zwischen der Brennpunktinformation (x, y, z) an dreidimensionalen Koordinaten und der Istwert-Brennpunktinformation (x₁, y₁, z₁) an den dreidimensionalen Koordinaten.

7. Medizinische Ultraschallvorrichtung nach Anspruch 6, wobei die Kompensationseinheit ausgelegt ist
zum Erzeugen, wenn die zweite Bilddarstellung das zweidimensionale Bild ist, der Kompensationswertinformation (x + Δx, z + Δz) durch Anwenden des Kompensationswerts (Δx, Δz) auf die Brennpunktinformation (x, z) an den zweidimensionalen Koordinaten, und
zum Erzeugen, wenn die zweite Bilddarstellung das dreidimensionale Bild ist, der Kompensationswertinformation (x + Δx, y + Δy, z + Δz) durch Anwenden des Kompensationswerts (Δx, Δy, Δz) auf die Brennpunktinformation (x, y, z) an den dreidimensionalen Koordinaten.

8. Medizinische Ultraschallvorrichtung nach Anspruch 1, wobei die Benutzereingabeeinheit ausgelegt ist
zum Empfangen, wenn die erste Bilddarstellung ein zweidimensionales Bild ist, einer Eingabe der Brennpunktinformation zweidimensionaler Koordinaten (x, z), und
zum Empfangen, wenn die erste Bilddarstellung ein dreidimensionales Bild ist, einer Eingabe der Brennpunktinformation dreidimensionaler Koordinaten (x, y, z).

9. Medizinische Ultraschallvorrichtung nach Anspruch 1, wobei der Behandlungswandler aufweist:
mindestens einen ersten Wandler, ausgelegt zum Senden eines ersten Ultraschalls hoher Intensität zum Brennpunkt, aufweisend eine Intensität, die geringer ist als ein vorbestimmter Ausgabewert mit Bezug auf eine Grundfrequenz,
mindestens einen zweiten Wandler, ausgelegt zum Senden eines zweiten Ultraschalls hoher Intensität zum Brennpunkt, aufweisend eine Intensität, die geringer ist als ein vorbestimmter Ausgabewert mit Bezug auf eine zweite Frequenz,
mindestens einen N-ten Wandler, ausgelegt zum Senden eines N-ten Ultraschalls hoher Intensität zum Brennpunkt, aufweisend eine Intensität, die geringer ist als ein vorbestimmter Ausgabewert mit Bezug auf eine N-te Frequenz.

10. Medizinische Ultraschallvorrichtung nach Anspruch 9, wobei der Behandlungswandler aufweist:
eine erste Gruppe bestehend aus den ersten Wandlern,
eine zweite Gruppe bestehend aus den zweiten Wandlern, und
eine N-te Gruppe bestehend aus den N-ten Wandlern.

11. Medizinische Ultraschallvorrichtung nach Anspruch 9, wobei der Behandlungswandler die ersten Wandler, die zweiten Wandler und die N-ten Wandler nach dem Zufallsprinzip angeordnet aufweist.

## Revendications

1. Appareil médical ultrasonore (100), comprenant :
un transducteur d'imagerie (110) conçu pour :
émettre un premier ultrason d'imagerie vers un objet cible,
recevoir un premier signal d'écho réfléchi par l'objet cible en réponse au premier ultrason d'imagerie,
générer un premier signal de réception sur la base du premier signal d'écho,
émettre un second ultrason d'imagerie vers l'objet cible,
recevoir un second signal d'écho réfléchi par l'objet cible en réponse au second ultrason d'imagerie, et
générer un second signal de réception sur la base du second signal d'écho ;
une unité de traitement de signaux (134) conçue pour :
générer une première image sur la base du premier signal de réception,
sortir la première image vers une unité d'affichage, et
générer une seconde image sur la base du second signal de réception ;
une unité d'entrée utilisateur (150) conçue pour recevoir une entrée d'informations de point focal sur la première image ;
un transducteur de traitement (112) conçu pour émettre un premier ultrason haute intensité pour préciblage ayant une intensité inférieure à une valeur de sortie prédéfinie et pour émettre un second ultrason haute intensité pour traitement ayant une intensité correspondant à la valeur de sortie prédéfinie vers un point focal correspondant aux informations de point focal ;
dans lequel le transducteur d'imagerie et le transducteur de traitement sont synchronisés de sorte que le transducteur d'imagerie soit conçu pour émettre le second ultrason d'imagerie vers l'objet cible lorsque le premier ultrason haute intensité atteint le point focal, de sorte que la seconde image reflète une variation de densité de l'objet cible en résultat au premier ultrason haute intensité ;
une unité d'extraction d'emplacement de focalisation (138) conçue pour :
extraire de la seconde image un emplacement présentant une plus grande variation d'échelle de gris par rapport à la première image, et
dans lequel l'emplacement extrait représente des informations de point focal réel représentant un point focal réel au niveau duquel est focalisé le premier ultrason haute intensité ; et
une unité de compensation (139) conçue pour :
générer une valeur de compensation basée sur les informations de point focal et les informations de point focal réel, et
appliquer la valeur de compensation aux informations de point focal.

2. Appareil médical ultrasonore selon la revendication 1, dans lequel le transducteur de traitement est conçu pour émettre le premier ultrason haute intensité ayant un cycle d'utilisation inférieur à un rapport prédéfini et une amplitude d'impulsion dépassant une valeur seuil prédéfinie.

3. Appareil médical ultrasonore selon la revendication 2, dans lequel le transducteur de traitement est conçu pour réaliser des émissions à répétition prédéfinie du premier ultrason haute intensité.

4. Appareil médical ultrasonore selon la revendication 1, dans lequel l'unité de traitement de signaux est conçue
pour recevoir le premier signal d'écho du transducteur d'imagerie qui est mis à tourner selon un angle prédéfini dans une plage de 0 degré à 360 degrés, ou
pour recevoir le premier signal d'écho du transducteur d'imagerie comprenant un réseau de transducteurs bidimensionnels, et
pour générer la première image en tant qu'image tridimensionnelle basée sur le premier signal d'écho.

5. Appareil médical ultrasonore selon la revendication 1, dans lequel l'unité d'extraction d'emplacement de focalisation est conçue
pour déterminer, lorsque la seconde image est une image bidimensionnelle, des coordonnées bidimensionnelles de l'emplacement ayant la plus grande quantité de variation de l'échelle de gris de la seconde image en tant qu'informations de point focal réel (x₁, z₁), et
pour déterminer, lorsque la seconde image est une image tridimensionnelle, des coordonnées tridimensionnelles de l'emplacement ayant la plus grande quantité de variation de l'échelle de gris de la seconde image en tant qu'informations de point focal réel (x₁, y₁, z₁).

6. Appareil médical ultrasonore selon la revendication 1, dans lequel l'unité de compensation est conçue
pour générer, lorsque la seconde image est une image bidimensionnelle, la valeur de compensation (Δx, Δz) sur la base d'une différence (x - x₁, z - z₁) entre les informations de point focal (x, z) sur des coordonnées bidimensionnelles et les informations de point focal réel (x₁, z₁) sur les coordonnées bidimensionnelles, et
pour générer, lorsque la seconde image est une image tridimensionnelle, la valeur de compensation (Δx, Δy, Δz) sur la base d'une différence (x - x₁, y - y₁, z - z₁) entre les informations de point focal (x, y, z) sur des coordonnées tridimensionnelles et les informations de point focal réel (x₁, y₁, z₁) sur les coordonnées bidimensionnelles.

7. Appareil médical ultrasonore selon la revendication 6, dans lequel l'unité de compensation est conçue
pour générer, lorsque la seconde image est l'image bidimensionnelle, des informations d'emplacement de compensation (x + Δx, z + Δz) en appliquant la valeur de compensation (Δx, Δz) aux informations de point focal (x, z) sur les coordonnées bidimensionnelles, et
pour générer, lorsque la seconde image est l'image tridimensionnelle, des informations d'emplacement de compensation (x + Δx, y + Δy, z + Δz) en appliquant la valeur de compensation (Δx, Δy, Δz) aux informations de point focal (x, y, z) sur les coordonnées tridimensionnelles.

8. Appareil médical ultrasonore selon la revendication 1, dans lequel l'unité d'entrée d'utilisateur est conçue
pour recevoir, lorsque la première image est une image bidimensionnelle, une entrée des informations de point focal de coordonnées bidimensionnelles (x, z), et
pour recevoir, lorsque la première image est une image tridimensionnelle, une entrée des informations de point focal de coordonnées tridimensionnelles (x, y, z) .

9. Appareil médical ultrasonore selon la revendication 1, dans lequel le transducteur de traitement comprend :
au moins un premier transducteur conçu pour émettre un premier ultrason haute intensité possédant une intensité inférieure à une valeur de sortie prédéfinie par rapport à une fréquence fondamentale vers le point focal,
au moins un second transducteur conçu pour émettre un second ultrason haute intensité possédant une intensité inférieure à une valeur de sortie prédéfinie par rapport à une fréquence secondaire vers le point focal, et
au moins un énième transducteur conçu pour émettre un énième ultrason haute intensité possédant une intensité inférieure à une valeur de sortie prédéfinie par rapport à une énième fréquence fondamentale vers le point focal.

10. Appareil médical ultrasonore selon la revendication 9, dans lequel le transducteur de traitement comprend :
un premier groupe constitué des premiers transducteurs,
un deuxième groupe constitué des deuxièmes transducteurs, et
un énième groupe constitué des énièmes transducteurs.

11. Appareil médical ultrasonore selon la revendication 9, dans lequel le transducteur de traitement comprend les premiers transducteurs, les deuxièmes transducteurs et les énièmes transducteurs agencés de façon aléatoire.
